# EUROPÄISCHE PATENTSCHRIFT

(11) **EP 0 967 061 B1**
(45) Veröffentlichungstag und Bekanntmachung des Hinweises auf die Patenterteilung: **02.03.2005**
(21) Anmeldenummer: 99105713.4
(22) Anmeldetag: 20.03.1999
(51) Int. Cl.: B29C 45/00, B23K 26/00, A61F 9/02, B29K 269/00

(54) **Verfahren zur Herstellung einer Skibrille**
Process for making a ski-goggle
Procédé pour la Fabrication des lunettes de ski

(30) Priorität: 25.06.1998 DE 19828360
(43) Veröffentlichungstag der Anmeldung: 29.12.1999
(73) Patentinhaber: Uvex Arbeitsschutz GmbH, 90766 Fürth (DE)
(72) Erfinder: Mestermann, Heinz, 90584 Allersberg (DE); Hirschmann, Peter, Dr., 90768 Fürth (DE); Herrmann, Herbert, 86736 Auhausen (DE)
(74) Vertreter: Schneck, Herbert, Dipl.-Phys., Dr.

(56) Entgegenhaltungen:
- DE-A- 3 734 656
- DE-A- 4 225 680
- US-A- 4 043 637
- US-A- 5 216 759
- US-A- 5 328 816
- US-A- 5 455 129
- US-A- 5 548 098

## Beschreibung

Die Erfindung richtet sich auf ein Verfahren zur Herstellung einer Skibrille mit einer Scheibe aus Polycarbonat, die längs ihrer Außenkontur in einen Rahmen eingesetzt wird.

Scheiben von Skibrillen bestehen meistens aus Acetat oder Zelluloseproprionat, wobei die Scheiben aus einem Plattenmaterial ausgestanzt werden. Derartige Acetat-Scheiben weisen den Nachteil auf, daß sie, insbesondere bei tieferen Temperaturen, relativ leicht brechen und bei einem Sturz Verletzungen im Gesicht des Skifahrers verursachen können.

Bei Arbeitsschutzbrillen, Sportbrillen und vereinzelt auch Skibrillen ist es auch schon bekannt, als Scheibenmaterial Polycarbonat zu verwenden, welches eine höhere Bruchfestigkeit aufweist, sich gut mit einer Antikratz- und einer Antibeschlagbeschichtung versehen läßt und auch - wenn solche Scheiben gespritzt werden - die Möglichkeiten einer optischen Korrektur eröffnet. Allerdings sind Polycarbonat-Scheiben bisher teurer und aufwendiger in der Verarbeitung.

Der Rahmen von Brillen mit Polycarbonat-Scheiben kann aus thermoplastischem Polyurethan oder thermoplastischen Elastomeren bestehen, wobei diese Materialien die an sich wünschenswerten Eigenschaften nicht in optimaler Weise besitzen. Sie haben allerdings den Vorteil, daß aus diesen Materialien keine Weichmacher in die Scheibe eindringen, mit der sie ja in engem Kontakt stehen, so daß eine durch Weichmacher bedingte Spannungsrißkorrosion der Polycarbonat-Scheiben bei diesen Materialien verhindert wird.

Bei Skibrillen mit Acetat-Scheiben werden Rahmen aus Polyvinylchlorid eingesetzt.

In der US 4,043,637 ist eine Skibrille offenbart, die einen Rahmen und eine aus Polycarbonat bestehende photochrome Scheibe aufweist. Die Scheibe steht mit dem Rahmen in Eingriff. Ein Verfahren zur Herstellung der Scheibe ist in dieser Druckschrift außerdem angegeben.

Eine Arbeitsschutzbrille mit einem Rahmen aus Polyvinylchlorid (PVC) und einer eingesetzten Scheibe aus Polycarbonat ist aus der US 5,216,759 bekannt. Die Kombination einer Polycarbonat-Scheibe mit einem PVC-Rahmen weist den gravierenden Nachteil auf, dass aus dem PVC-Material Weichmacher in die Scheibe dringt. Dies hat eine kurze Lebensdauer der Arbeitsschutzbrille wegen einer Spannungsrisskorrosion der Scheibe zur Folge.

Hiervon ausgehend liegt der Erfindung die Aufgabe zugrunde, ein Verfahren zur Herstellung einer Skibrille mit einer derartigen Scheibe zu schaffen, welche eine hohe Bruchsicherheit mit einer hohen Korrosionsbeständigkeit und hervorragende Trageeigenschaften sowie ein optisch ansprechendes Erscheinungsbild aufweist.

Diese Aufgabe wird gelöst durch ein Verfahren der eingangs genannten Art, welches sich dadurch auszeichnet, dass die Scheibe durch Laser-Schneiden aus einem gespritzten Scheiben-Rohling ausgeschnitten wird, wobei die Oberfläche durch Schmelzen geschlossen wird, und dass der Rahmen aus Weich-Polyvinylchlorid besteht.

Erfindungsgemäß werden mikroskopische Oberflächenbeschädigungen, wie sie z.B. beim Stanzen oder beim Schneiden mit anderen Werkzeugen entstehen, vermieden. Dementsprechend können diese Oberflächenbeschädigungen nicht als Ausgangspunkte von Spannungsriß-Korrosionen wirken und es wird auch eine Förderung dieser Korrosion durch die Migration von Weichmachern vermieden, da der Randbereich der Scheibe durch Aufschmelzen vollständig geschlossen ist.

Durch die Verwendung von Polycarbonat-Scheiben mit geschlossenen Randbereichen ist es überraschenderweise möglich, den Rahmen aus PVC herzustellen, wobei sich PVC im Gegensatz zu herkömmlicherweise fur den Rahmen von Brillen mit Polycarbonat-Scheiben verwendeten Materialien leichter verarbeiten läßt, als Rohmaterial preiswerter ist und eine ansprechendere Gestaltung des optischen Erscheinungsbildes ermöglicht. Die Kombination ermöglicht also sowohl eine Optimierung der Materialauswahl für den Rahmen als auch für die eigentliche Scheibe, die sich insbesondere durch ihre hohe Bruchsicherheit auszeichnet, was gerade bei Skibrillen von besonderer Bedeutung ist.

Nachfolgend wird die Erfindung anhand eines bevorzugten Ausführungsbeispiels in Verbindung mit der Zeichnung näher erläutert. Dabei zeigen:
Fig. 1 einen Scheiben-Rohling und
Fig. 2 eine blockschaltbildartige Darstellung einer Anlage zur Durchführung des erfindungsgemäßen Verfahrens.

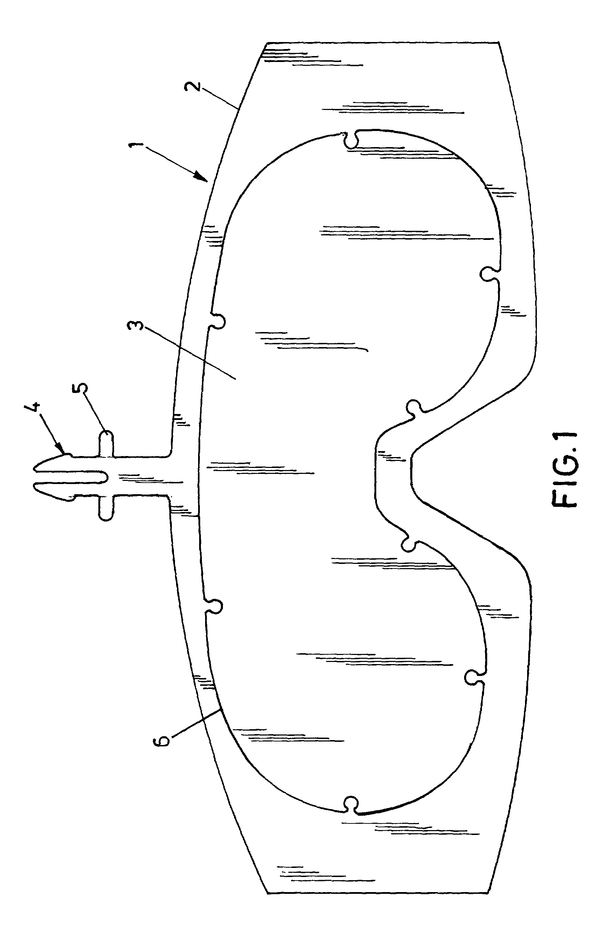

Ein in Fig. 1 dargestellter Scheiben-Rohling 1 weist eine Außenkontur 2 auf, die deutlich größer ist als die herzustellende Scheibe 3, wobei sich von dieser Außenkontur 2 ein Ansatz 4 wegerstreckt mit einem Quersteg 5, so daß die Scheiben-Rohlinge 1 mittels dieses Quersteges 5 auf einer Schiene geführt der Bearbeitung, d.h. dem Laser-Schneiden, zugeführt werden können.

Die Scheiben-Rohlinge 1 werden an einem stationären Laser mittels einer CNC-Steuerung so vorbeigeführt, daß unter Schutzgasatmosphäre in den Scheiben-Rohling 1 die Scheibenkontur 6 der gebrauchsfertigen Scheibe eingeschnitten wird.

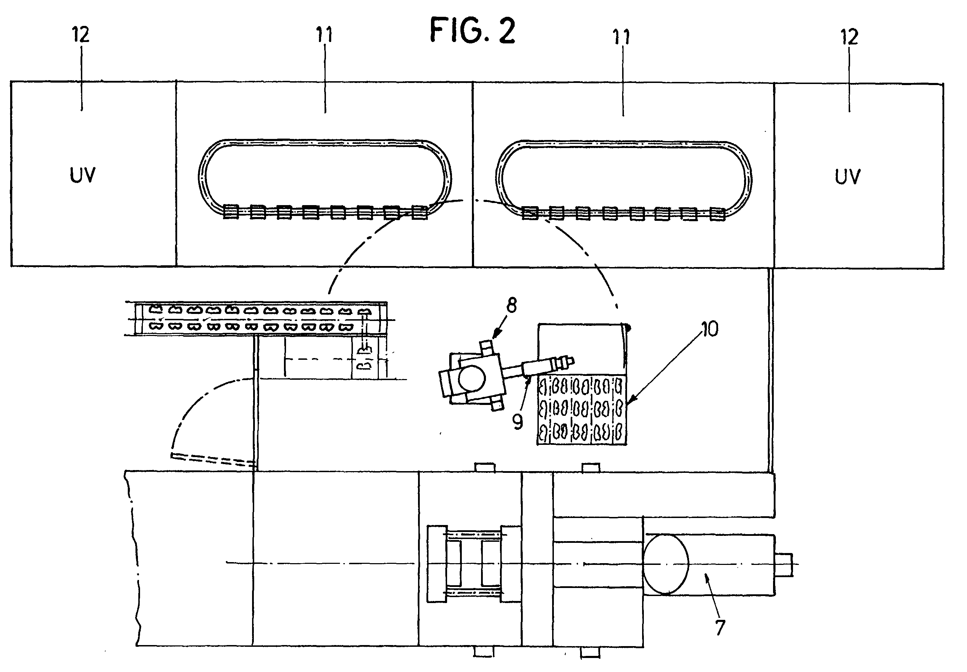

Der in Fig. 1 dargestellte Scheiben-Rohling 1 wird mit einem im Zusammenhang mit Fig. 2 nachfolgend näher beschriebenen Verfahren hergestellt:

In Fig. 2 ist ein Spritzgießautomat 7 dargestellt, in welchem Scheiben-Rohlinge 1 gespritzt, ausgeworfen und von einem Industrieroboter 8 mit einem Greifarm aufgenommen und einer Abkühlstation 10 zugeführt werden.

Dort wird eine Mehrzahl von Scheiben zum Abkühlen zwischengelagert, indem der Roboter 8 eine Scheibe, die soeben gespritzt wurde, an einem freien Platz ablegt und diejenige Scheibe wieder aufnimmt, die am längsten in der Abkühlstation 10 gelegen hatte.

Jenseits der Spritzgießmaschine 7 und des Industrieroboters 8 sind zwei Beschichtungsanlagen 11 vorgesehen, wobei der Roboter 8 des Beschichtungsanlagen 11 abwechselnd Scheiben-Rohlinge 1 zuführt, die von der Abkühlstation 10 entnommen werden.

Die Scheiben-Rohlinge 1 werden in den Beschichtungsanlagen 11 im Oval transportiert, wobei der Transport schrittweise erfolgt, so daß in einer ersten Beschichtungseinrichtung beispielsweise längs der Oberkante der Scheiben-Rohlinge 1 eine kratzfeste Beschichtung ausbildender Lack auf die Vorderseite aufgebracht wird, in einer zweiten Beschichtungseinrichtung ein ein Beschlagen verhindernder Lack auf die Rückseite aufgebracht wird, wobei die so beschichteten Scheiben-Rohlinge 1 dann anschließend in einer UV-Station 12 mittels UV-Licht getrocknet und gehärtet werden.

Vom Vorgang des Spritzens bis zum Härten verläuft alles in einem einzigen, ununterbrochenen Reinraumbereich, so daß die Scheiben-Rohlinge 1 während des Herstellungsvorganges nicht verschmutzen können und auch eine Zwischenlagerung und eine eventuelle Reinigung nach der Zwischenlagerung nicht erforderlich werden.

## Patentansprüche

1. Verfahren zur Herstellung einer Skibrille mit einer Scheibe (3) aus Polycarbonat, die längs ihrer Außenkontur (2) in einen Rahmen eingesetzt wird, **dadurch gekennzeichnet, dass** die Scheibe (3) durch Laser-Schneiden aus einem gespritzten Scheiben-Rohling (1) ausgeschnitten wird, wobei die Oberfläche durch Schmelzen geschlossen wird, und dass der Rahmen aus Weich-Polyvinylchlorid besteht.

## Claims

1. Method for the manufacture of skiing goggles, comprising a sight piece (3) of polycarbonate which is inserted into a frame along its outer contour (2), **characterized in that** the sight piece (3) is cut from an injection-molded blank (1) by laser-beam cutting, with the surface being closed by melting; and **in that** the frame consists of soft polyvinyl chloride.

## Revendications

1. Procédé pour la fabrication de lunettes de ski avec un verre (3) en polycarbonate qui est inséré dans un châssis le long de son contour extérieur (2),
**caractérisé en ce que**,
le verre (3) est découpé au laser à partir d'une ébauche de verre coulée par injection (1), tandis que la surface est fermée par fusion et **en ce que** le châssis est constitué de chlorure de polyvinyle mou.
